# EUROPÄISCHE PATENTSCHRIFT

(11) **EP 0 638 369 B1**
(45) Veröffentlichungstag und Bekanntmachung des Hinweises auf die Patenterteilung: **03.03.1999**
(21) Anmeldenummer: 94108763.7
(22) Anmeldetag: 08.06.1994
(51) Int. Cl.: B07C 5/36, B08B 9/46, G01N 21/90

(54) **Inspektionsmaschine für Flaschen oder dgl.**
Machine for inspecting bottles or analogues
Machine pour l'inspection de bouteilles ou autres récipients

(30) Priorität: 16.07.1993 DE 9310623 U
(43) Veröffentlichungstag der Anmeldung: 15.02.1995
(73) Patentinhaber: KRONES AG, 93068 Neutraubling (DE)
(72) Erfinder: Häring, Franz, D-93073 Neutraubling (DE); Griesbeck, Karl, D-93053 Regensburg (DE)

(56) Entgegenhaltungen:
- EP-A- 0 119 681
- EP-A- 0 415 154
- EP-A- 0 489 267
- EP-B- 0 124 164
- DE-U- 8 811 597
- GB-A- 1 455 569
- US-A- 4 691 231
- US-A- 4 915 237

## Beschreibung

Die Erfindung betrifft eine Inspektionsmaschine für Flaschen oder dgl. gemäß dem Oberbegriff des Anspruchs 1.

Eine derartige Geradläufer-Inspektionsmaschine ist aus der EP 124 164 B1 bekannt. Bei dieser Inspektionsmaschine schließt sich an das Ende des durch ein Förderband gebildeten ersten Geradförderers ein Sammelbehälter an, in den z.B. liegende Flaschen, Scherben und zu niedrige Flaschen, die durch den von zwei unterhalb des Flaschenkopfs angreifenden, gegenläufig antreibbaren Riemen gebildeten zweiten Geradförderer nicht erfaßt werden, hineinfallen können. Hierbei besteht insbesondere bei höheren Transportgeschwindigkeiten die Gefahr, dass die zu niedrigen Flaschen usw. vom Ende des ersten Geradförderers über den Sammelbehälter hinweggeschleudert werden und die nachfolgende Bodeninspektionseinrichtung beschädigen. Zu große Flaschen können auf diese Weise überhaupt nicht ausgeschieden werden und können an den Inspektionseinrichtungen am zweiten Geradförderer schwerwiegende Beschädigungen verursachen.

Weiter ist eine Rundläufer-Inspektionsmaschine mit einer Mündungsinspektionseinrichtung an einem rotierenden Rundtisch und mit einer diesem vorgeordneten Einlaufschnecke bekannt, bei der an dem zur Einlaufschnecke führenden Förderband eine separate Bodeninspektionseinrichtung und eine von dieser steuerbare Ausleiteinrichtung angeordnet ist (DE-U-88 11 597). Diese bekannte Rundläufer-Inspektionsmaschine ist aufgrund der Aneinanderreihung der verschiedenen Inspektionseinrichtungen und deren Transport- und Ausleiteinrichtungen sehr raumgreifend aufgebaut. Eine Ausscheidung von Fremdflaschen, die Störungen an der Mündungsinspektionseinrichtung verursachen könnten, ist nicht vorgesehen.

Schließlich ist bereits eine Rundläufer-Füllmaschine für Flaschen oder dgl. mit einem rotierenden Rundtisch und einem vorgeschalteten Einlaufstern bekannt, bei dem am Einlaufstern eine Überwachungseinrichtung auf Fremdflaschen angeordnet ist (GB-A- 1 455 569). Die erkannten Fremdflaschen werden durch mit dem Einlaufstern umlaufende, steuerbare Greifzangen vorübergehend festgehalten und somit nicht an den Rundtisch übergeben.

Der Erfindung liegt die Aufgabe zugrunde, bei einer Inspektionsmaschine für Flaschen oder dgl. der eingangs genannten Art die Ausscheidung von Fremdflaschen zu verbessern.

Diese Aufgabe wird erfindungsgemäß durch die im Kennzeichen des Anspruchs 1 angegebenen Merkmale gelöst.

Bei einer erfindungsgemäßen Inspektionsmaschine können je nach Ausgestaltung der Überwachungseinrichtung mehr oder weniger viele Arten von Fremdflaschen, bis hin zu Flaschen mit falscher Farbe und/oder falscher Kontur, insbesondere aber auch mit zu großer Höhe, sicher erkannt und dann durch die Ausleiteinrichtung zuverlässig seitlich ausgeschieden werden. Auf diese Weise wird jegliche Gefahr der Beschädigung des zweiten Geradförderers und der an diesem angeordneten Inspektionseinrichtungen vollständig eliminiert. Zwischen dem Ende des ersten Geradförderers und der ersten Inspektionseinrichtung am zweiten Geradförderer ist kein nennenswerter Zwischenraum erforderlich, so dass sich die Baulänge der Inspektionsmaschine wesentlich verkürzt. Durch die Anordnung der Wandinspektionseinrichtung am ersten Geradförderer hat dieser einen mehrfachen Nutzen. Die üblichen Wandinspektionseinrichtungen mit einer Beleuchtungseinrichtung auf der einen und mindestens einer elektronischen Kamera auf der anderen Seite der Bewegungsbahn der Flaschen sind weitgehend unempfindlich gegen Fremdflaschen, so dass eine Anordnung der Ausleiteinrichtung für die Fremdflaschen im Anschluß an die Wandinspektionseinrichtung keine negativen Auswirkungen hat. Schließlich trägt auch die Integration der Überwachungseinrichtung auf Fremdflaschen in die Wandinspektionseinrichtung zu einer kurzen, kompakten Bauweise bei.

Für den Aufbau und die Zuordnung der beiden Linearförderer gibt es verschiedene Möglichkeiten, wovon einige in den Ansprüchen 2 bis 5 angegeben sind.

Besonders vorteilhaft ist es, wenn gemäß Anspruch 6 am Einlauf des ersten Geradförderers eine die Flaschen auf Abstand bringende Zuführeinrichtung, z.B. ein langsamer laufendes Band, ein Transportstern oder eine Förderschnecke, angeordnet ist. Der dadurch bewirkte Abstand zwischen den aufeinanderfolgenden Flaschen erleichtert sowohl die Kontrolle der Flaschen in der Überwachungseinrichtung als auch die Ausscheidung in der Ausleitvorrichtung.

Für den Aufbau der Ausleiteinrichtung gibt es verschiedene Möglichkeiten. Ein besonders einfacher und zuverlässiger Aufbau wird erzielt, wenn gemäß Anspruch 10 die Ausleiteinrichtung einen impulsartig steuerbaren Stößel aufweist. Dieser arbeitet zweckmäßigerweise mit einer Öffnung im Führungsgeländer und einem Sammelbehälter zusammen, wie dies im Anspruch 11 und 12 angegeben ist.

Sollen die Fremdflaschen auf ein eigenes, vom ersten Geradförderer weggerichtetes Förderband oder dgl. überführt werden, so ist ein etwas aufwendigerer Aufbau der Ausleiteinrichtung, beispielsweise durch mit den Gefäßen mitlaufende, kurvengesteuerte Stößel, zweckmäßig.

Im Nachstehenden wird ein Ausführungsbeispiel der Erfindung anhand der Zeichnungen beschrieben. Es zeigen:
- Fig. 1: die Draufsicht und
- Fig. 2: die Seitenansicht einer Inspektionsmaschine für Flaschen.

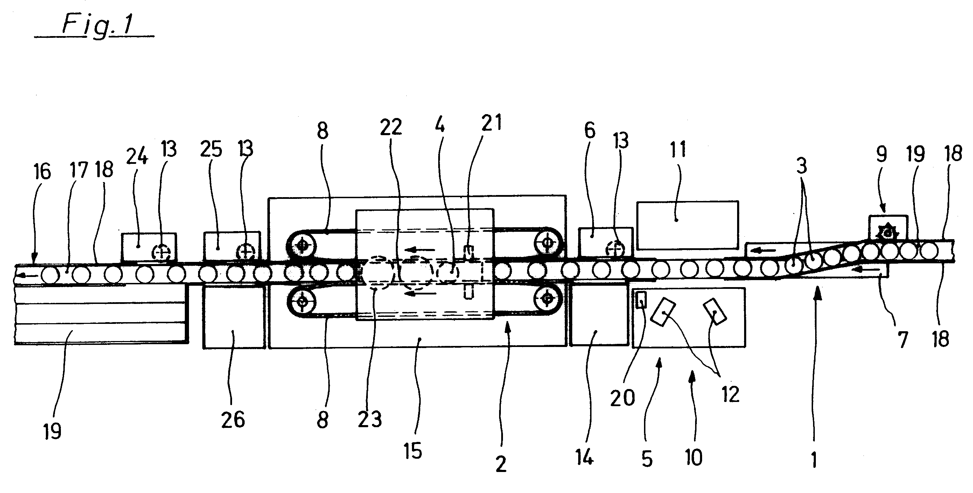

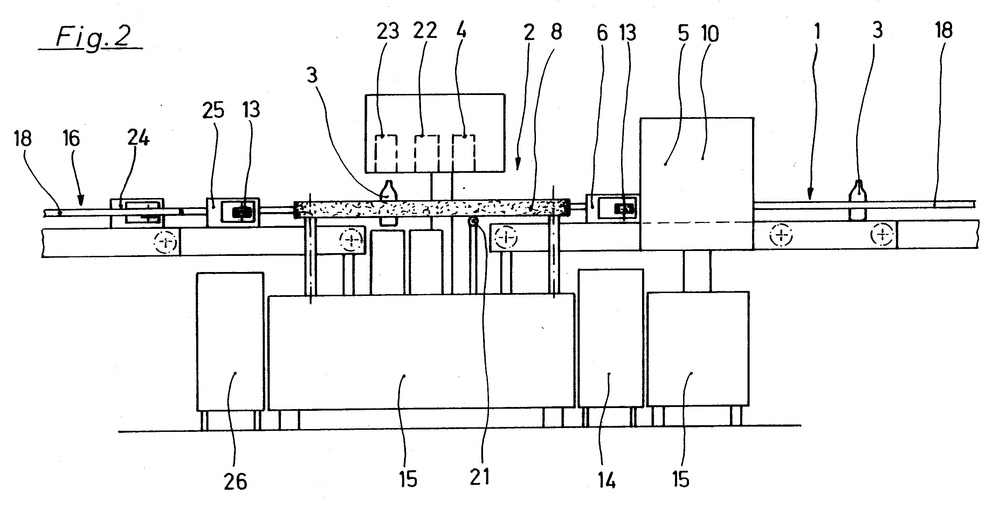

Die Inspektionsmaschine nach Fig. 1 und 2 ist zum Überprüfen von gereinigten Mehrwegflaschen aus Glas für Getränke, im Nachstehenden kurz Flaschen 3 genannt, auf Verschmutzungen und Fremdkörper im Wand- und Bodenbereich sowie auf Laugenreste im Flascheninneren eingerichtet. Sie ist dementsprechend in eine nicht weiter gezeigte Flaschenabfüllanlage zwischen der Reinigungsmaschine und der Füll- und Verschließmaschine integriert.

Die Inspektionsmaschine weist ein zweiteiliges Gehäuse 15 auf, in dem verschiedene Antriebs- und Steuereinrichtungen untergebracht sind. An der Oberseite des Gehäuses 15 sind ein erster Geradförderer 1, ein zweiter Geradförderer 2 und dritter Geradförderer 16 derart fluchtend angeordnet, daß die Flaschen 3, genauer gesagt die einwandfreien Flaschen 3, auf einer durchgehend geradlinigen Bahn die Inspektionsmaschine durchlaufen.

Der erste Geradförderer 1 und der dritte Geradförderer 16 weisen jeweils ein endloses Förderband 7 bzw. 17 in Form einer Scharnierbandkette auf, das über in einem Profilrahmen gelagerte Antriebs- und Umlenkräder umläuft. Die Förderbänder 7, 17 liegen in einer gemeinsamen horizontalen Ebene und weisen an den erforderlichen Bereichen seitliche Führungsschienen 18 auf, die auf den Durchmesser der zu prüfenden Flaschen 3 eingestellt sind. Die Flaschen 3 werden somit auf dem ersten Geradförderer 1 und dem dritten Geradförderer 16 aufrechtstehend mit Bodenunterstützung in einer geraden Einzelreihe in Pfeilrichtung transportiert.

Zwischen dem ersten Geradförderer 1 und dem dritten Geradförderer 16 ist eine Lücke von mehreren Flaschendurchmessern vorhanden, die durch den zweiten Geradförderer 2 überbrückt wird. Dieser weist zwei endlose, antreibbare Riemen 8 auf, die in einer gemeinsamen horizontalen Ebene etwas überhalb der Ebene der Förderbänder 7 und 17 mit gegenseitigem Abstand und entgegengesetztem Drehsinn in Pfeilrichtung umlaufen. Die Riemen 8 sind an ihrer Außenseite mit einem elastischen Reibungsbelag versehen und derart beabstandet, daß sie Flaschen 3 der zu prüfenden Sorte im Rumpfbereich zwischen sich einklemmen und in aufrechter Haltung hängend, d.h. ohne Bodenunterstützung, über die Lücke zwischen dem ersten Geradförderer 1 und dem dritten Geradförderer 16 hinweg transportieren. Hierzu überdeckt der Anfangsbereich des zweiten Geradförderers 2 den Endbereich des ersten Geradförderers 1 und der Endbereich des zweiten Geradförderers 2 den Anfangsbereich des dritten Geradförderers 16. Außerdem werden die drei Geradförderer 1, 2, 16 durch nicht gezeigte Antriebselemente synchron mit der gleichen Translationsgeschwindigkeit angetrieben.

Zur Zuführung der zu prüfenden Flaschen 3 zur Inspektionsmaschine ist dem ersten Geradförderer 1 ein weiteres Förderband 19 mit seitlichen Führungsschienen 18 vorgeschaltet. Dieses ist synchron zum ersten Geradförderer 1 mit einer etwas langsameren Geschwindigkeit angetrieben, so daß die Flaschen 3 während der Überführung vom Förderband 19 auf das Förderband 7 durch die entsprechend schräggestellten Führungsschienen 18 etwas beschleunigt und auf einen bestimmten Abstand gebracht werden. Vor dem Überschubbereich ist ein frei drehbarer Sperrstern angeordnet, der in gewissen Betriebszuständen blockiert werden kann, um die weitere Zufuhr von Flaschen 3 zur Inspektionsmaschine zu unterbrechen.

Zu beiden Seiten des ersten Geradförderers 1 ist eine Wandinspektionseinrichtung 5 angeordnet und zwar mit einem gewissen Abstand vor dem Anfangsbereich des zweiten Geradförderers 2. Die Wandinspektionseinrichtung 5 weist einerseits eine Beleuchtungseinrichtung 11 und andererseits zwei elektronische Videokameras 12 auf, die von jeder vorbeilaufenden Flasche 3 zwei Bilder aufnehmen. Anhand dieser Bilder wird durch eine elektronische Auswertungseinrichtung zum einen entschieden, ob es sich um eine ordnungsgemäße Flasche 3 der abzufüllenden Sorte oder um eine Fremdflasche handelt, und zum anderen, ob die Flasche 3 am Umfang mit Verunreinigungen versehen ist oder nicht. Fremdflaschen können z.B. durch abweichende Kontur oder abweichende Höhe erkannt werden. Erforderlichenfalls kann zusätzlich ein Farbsensor 20 vorgesehen werden, damit auch Flaschen 3 mit übereinstimmender Kontur und Höhe, jedoch unzulässiger Farbe erkannt werden.

Die erkannten Fremdflaschen werden durch eine Ausleiteinrichtung 6, die zwischen der Wandinspektionseinrichtung 10 und dem Anfangsbereich des zweiten Geradförderers 2 am ersten Geradförderer 1 angeordnet ist, seitlich ausgestoßen. Zu diesem Zweck ist an der der Ausleiteinrichtung 6 gegenüberliegenden Seite des ersten Geradförderers 1 eine Lücke in den Führungsschienen 18 ausgebildet und ein Sammelbehälter 14 aufgestellt. Die ausgeschiedenen Fremdflaschen gelangen somit nicht in den zweiten Geradförderer 2 und können dort keinen Schaden anrichten. Die Ausleiteinrichtung 6 weist eine in der Ruhestellung seitlich neben dem Förderband 7 sitzende Schwammrolle 13 auf, die beim Passieren einer Fremdflasche durch einen nicht gezeigten Pneumatikzylinder impulsartig in die Bewegungsbahn der Flaschen 3 hineinbewegt und wieder zurückgezogen wird und dabei die betreffende Fremdflasche gezielt in den Sammelbehälter 14 befördert.

Die in der Wandinspektionseinrichtung 10 erkannten Schmutzflaschen 3 der ordnungsgemäßen Flaschensorte laufen unbehelligt an der Ausleiteinrichtung 6 vorbei und werden von den Riemen 8 des zweiten Geradförderers 2 erfaßt und eingeklemmt, genauso wie die von der Wandinspektionseinrichtung 10 nicht beanstandeten Flaschen 3. Alle diese Flaschen 3 passieren mit dem zweiten Geradförderer 2 nacheinander eine oder mehrere Laugenkontrolleinrichtungen 21, eine erste Bodeninspektionseinrichtung 22 und eine zweite Bodeninspektionseinrichtung 23 und werden dann an den dritten Geradförderer 16 übergeben. An diesem ist eine weitere Ausleiteinrichtung 24 vom gleichen Aufbau wie die erste Ausleiteinrichtung 6 angeordnet, von der alle Flaschen 3, die in einer oder mehrerer der Inspektionsstationen 10, 21, 22 und 23 beanstandet worden sind, seitlich vom Förderband 17 weg auf einen durch mehrere angetriebene Förderbänder gebildeten Sammeltisch 19 geleitet werden.

Am zweiten Geradförderer 2 können weitere Kontrolleinrichtungen für die Flaschen 3 angeordnet werden, wie Z.B. eine Mündungs- und Gewindeinspektionseinrichtung 4. Diese arbeitet mit einer eigenen, am dritten Geradförderer 17 angeordneten Ausleiteinrichtung 25 der gleichen Bauart wie die erste Ausleiteinrichtung 6 zusammen. Durch die Ausleiteinrichtung 25 werden nur Flaschen 3 mit Beschädigung der Mündung und/oder des Gewindes in einen eigenen Sammelbehälter 26 ausgestoßen.

## Patentansprüche

1. Inspektionsmaschine für Flaschen oder dgl. mit einem ersten Geradförderer (1), der die Flaschen mit Bodenunterstützung in einer Reihe hintereinander transportiert, einem unmittelbar nachfolgenden zweiten Geradförderer (2), der die Flaschen ohne Bodenunterstützung in einer Reihe hintereinander durch eine Bodeninspektionseinrichtung (22, 23) transportiert, sowie mit einer Wandinspektionseinrichtung (10), dadurch gekennzeichnet, dass der erste Geradförderer (1) die Flaschen durch die Wandinspektionseinrichtung (10) transportiert, dass am ersten Geradförderer (1) eine zumindest teilweise in die Wandinspektionseinrichtung (10) integrierte Überwachungseinrichtung (5) auf Fremdflaschen angeordnet ist, und dass zwischen der Wandinspektionseinrichtung (10) und dem zweiten Geradförderer (2) eine von der Überwachungseinrichtung (5) steuerbare Ausleiteinrichtung (6) angeordnet ist, welche nur die erkannten Fremdflaschen noch vor Erreichen des zweiten Geradförderers (2) seitlich vom ersten Geradförderer (1) entfernt.

2. Inspektionsmaschine nach Anspruch 1, dadurch gekennzeichnet, dass der erste Geradförderer (1) mindestens ein antreibbares Förderband (7) aufweist, welches am Flaschenboden angreift.

3. Inspektionsmaschine nach Anspruch 1 oder 2, dadurch gekennzeichnet, dass der zweite Geradförderer (2) mindestens einen antreibbaren Riemen (8) aufweist, welcher am Flaschenumfang oder Flaschenkopf angreift.

4. Inspektionsmaschine nach einem der Ansprüche 1 bis 3, dadurch gekennzeichnet, dass die beiden Geradförderer (1, 2) in einer Flucht angeordnet sind, so dass die Flaschen durchgehend geradlinig transportiert werden.

5. Inspektionsmaschine nach einem der Ansprüche 1 bis 4, dadurch gekennzeichnet, dass der Anfangsbereich des zweiten Geradförderers (2) den Endbereich des ersten Geradförderers (1) überdeckt.

6. Inspektionsmaschine nach einem der Ansprüche 1 bis 5, dadurch gekennzeichnet, dass am oder vor dem ersten Geradförderer (1) vor der Überwachungseinrichtung (5) eine die Flaschen auf Abstand bringende Zuführeinrichtung (9) angeordnet ist.

7. Inspektionsmaschine nach einem der Ansprüche 1 bis 6, dadurch gekennzeichnet, dass die Überwachungseinrichtung (5) mindestens einen Höhensensor aufweist.

8. Inspektionsmaschine nach einem der Ansprüche 1 bis 7, dadurch gekennzeichnet, dass die Überwachungseinrichtung (5) mindestens einen Farbsensor (20) aufweist.

9. Inspektionsmaschine nach einem der Ansprüche 1 bis 8, dadurch gekennzeichnet, dass die Überwachungseinrichtung (5) mindestens eine Beleuchtungseinrichtung (11) und mindestens eine elektronische Kamera (12) aufweist.

10. Inspektionsmaschine nach einem der Ansprüche 1 bis 9, dadurch gekennzeichnet, dass die Ausleiteinrichtung (6) mindestens einen neben dem ersten Geradförderer (1) gelagerten Stößel (13) aufweist, der in die Bewegungsbahn der Flaschen verfahrbar ist.

11. Inspektionsmaschine nach Anspruch 10, dadurch gekennzeichnet, dass die dem Stößel (13) gegenüberliegende Stelle des ersten Geradförderers (1) seitlich offen bzw. frei von Flaschenführungselementen (18) ist.

12. Inspektionsmaschine nach Anspruch 10 oder 11, dadurch gekennzeichnet, dass an der dem Stößel (13) gegenüberliegenden Seite des ersten Geradförderers (1) ein Sammelbehälter (14) für ausgestoßene Fremdflaschen angeordnet ist.

13. Inspektionsmaschine nach einem der Ansprüche 1 bis 12, dadurch gekennzeichnet, dass dem zweiten Geradförderer (2) ein dritter Geradförderer (16) nachgeschaltet ist und an diesem eine Ausleiteinrichtung (24) für in der Bodeninspektionseinrichtung (22, 23) und in der Wandinspektionseinrichtung (10) erkannte Schmutzflaschen angeordnet ist.

14. Inspektionsmaschine nach Anspruch 13, dadurch gekennzeichnet, dass am zweiten Geradförderer (2) eine Mündungs- und/oder Gewindeinspektionseinrichtung (4) und am dritten Geradförderer (16) eine zweite Ausleiteinrichtung (25) für in der Mündungs- und/oder Gewindeinspektionseinrichtung (4) erkannte beschädigte Flaschen angeordnet ist.

## Claims

1. An inspection machine for bottles or the like, having a first linear conveyor (1) which conveys the bottles supported at their bottoms in a row one behind another, having a directly following second linear conveyor (2) which conveys the bottles, without support at their bottoms, in a row one behind another through a bottom inspection device (22, 23), and having a wall inspection device (10), characterised in that the first linear conveyor (1) conveys the bottles through the wall inspection device (10), that a monitoring device (5) for extraneous bottles, which is at least in part integrated in the wall inspection device (10), is disposed on the first linear conveyor (1), and that an ejection device (6) which can be controlled by the monitoring device (5) is disposed between the wall inspection device (10) and the second linear conveyor (2), which ejection device removes only recognised extraneous bottles laterally from the first linear conveyor (1) before they reach the second linear conveyor (2).

2. An inspection machine according to claim 1, characterised in that the first linear conveyor (1) has at least one conveyor belt (7) which can be driven and which acts on the bottle bottom.

3. An inspection machine according to claims 1 or 2, characterised in that the second linear conveyor (2) has at least one belt (8) which can be driven and which acts on the bottle circumference or bottle top.

4. An inspection machine according to any one of claims 1 to 3, characterised in that the two linear conveyors (1, 2) are disposed in alignment, so that the bottles are continuously conveyed linearly.

5. An inspection machine according to any one of claims 1 to 4, characterised in that the initial region of the second linear conveyor (2) overlaps the end region of the first linear conveyor (1).

6. An inspection machine according to any one of claims 1 to 5, characterised in that a feeder device (9) which provides a spacing between the bottles is disposed on or in front of the first linear conveyor (1), in front of the monitoring device (5).

7. An inspection machine according to any one of claims 1 to 6, characterised in that the monitoring device (5) comprises at least one height sensor.

8. An inspection machine according to any one of claims 1 to 7, characterised in that the monitoring device (5) comprises at least one colour sensor (20).

9. An inspection machine according to any one of claims 1 to 8, characterised in that the monitoring device (5) comprises at least one illuminating device (11) and at least one electronic camera (12).

10. An inspection machine according to any one of claims 1 to 9, characterised in that the ejection device (5) comprises at least one pusher (13) which is mounted near the first linear conveyor (1) and which can move into the path of movement of the bottles.

11. An inspection machine according to claim 10, characterised in that the opposite side of the first linear conveyor (1) to the pusher (13) is open at the side or is free from bottle guide elements (18).

12. An inspection machine according to claims 10 or 11, characterised in that a collection vessel (14) for ejected extraneous bottles is disposed on the opposite side of the first linear conveyor (1) to the pusher (13).

13. An inspection machine according to any one of claims 1 to 12, characterised in that a third linear conveyor (16) is disposed downstream of the second linear conveyor (2), and an ejection device (24) for soiled bottles which are identified in the bottom inspection device (22, 23) and in the wall inspection device (10) is disposed on said third linear conveyor.

14. An inspection machine according to claim 13, characterised in that a mouth- and/or screw thread inspection device (4) is disposed on the second linear conveyor (2) and a second ejection device (25) for bottles which are identified in the mouth- and/or screw thread inspection device (4) as being damaged is disposed on the third linear conveyor (16).

## Revendications

1. Machine d'inspection de bouteilles ou objets analogues, comportant un premier transporteur rectiligne (1) qui transporte les bouteilles alignées les unes derrière les autres et soutenues par le fond, un second transporteur rectiligne (2) suivant directement le premier et qui fait passer les bouteilles alignées les unes derrière les autres mais sans être soutenues par le fond, à travers un dispositif d'inspection du fond (22, 23), ainsi qu'un dispositif d'inspection des parois (10),
caractérisée en ce que
• le premier transporteur rectiligne (1) fait passer les bouteilles à travers le dispositif d'inspection des parois (10),
• le long du premier transporteur se trouve, intégré au moins en partie au dispositif d'inspection des parois (10), un dispositif de surveillance (5) décelant les bouteilles étrangères,
• entre le dispositif d'inspection (10) et le second transporteur rectiligne (2) se trouve un dispositif de déviation (6) commandé par le dispositif de surveillance et qui, avant qu'elles atteignent le second transporteur (2), éloigne latéralement du premier transporteur les seules bouteilles reconnues étrangères.

2. Machine d'inspection selon la revendication 1,
caractérisée en ce que
le premier transporteur rectiligne (1) comporte au moins une bande transporteuse (7) qui peut être entraînée et qui est en contact avec les fonds des bouteilles.

3. Machine d'inspection selon l'une des revendications 1 ou 2,
caractérisée en ce que
le second transporteur rectiligne (2) comprend au moins une courroie (8) qui peut être entraînée et qui est en contact avec la périphérie ou la tête des bouteilles.

4. Machine selon l'une des revendications 1 ou 3,
caractérisée en ce que
les deux transporteurs rectilignes (1, 2) sont alignés de sorte que les bouteilles sont transportées toujours en ligne directe.

5. Machine selon l'une des revendications 1 à 4,
caractérisée en ce que
la zone située au début du second transporteur (2) recouvre la zone située à la fin du premier transporteur (1).

6. Machine selon l'une des revendications 1 à 5,
caractérisée en ce que
sur ou avant le premier transporteur rectiligne (1), un dispositif d'amenée (9), espaçant les bouteilles, est monté en amont du dispositif de surveillance (5).

7. Machine selon l'une des revendications 1 à 6,
caractérisée en ce que
le dispositif de surveillance (5) comprend au moins un détecteur de hauteur.

8. Machine selon l'une des revendications 1 à 7,
caractérisée en ce que
le dispositif de surveillance (5) comprend au moins un détecteur de couleur (20).

9. Machine selon l'une des revendications 1 à 8,
caractérisée en ce que
le dispositif de surveillance (5) comprend au moins un dispositif d'éclairage (11) et au moins une caméra électronique (12).

10. Machine selon l'une des revendications 1 à 9,
caractérisée en ce que
le dispositif de déviation (6) comprend au moins un poussoir (13) monté à côté du premier transporteur rectiligne (1) et qui peut intervenir sur la piste de déplacement des bouteilles.

11. Machine selon la revendication 10,
caractérisée en ce que
la zone du premier transporteur (1) située en face du poussoir (13) est ouverte latéralement et dépourvue d'éléments de guidage des bouteilles (18).

12. Machine selon l'une des revendications 10 ou 11,
caractérisée en ce que
sur le côté du premier transporteur (1) situé à l'opposé du poussoir (13) est placé un récipient collecteur (14) destiné aux bouteilles étrangères éjectées.

13. Machine selon l'une des revendications 1 à 12,
caractérisée en ce que
en aval du second transporteur rectiligne (2) est placé un troisième transporteur rectiligne (16) sur lequel est monté un dispositif de déviation (24) destiné à évacuer les bouteilles reconnues sales par le dispositif d'inspection des fonds (22, 23) et par le dispositif d'inspection des parois (10).

14. Machine selon la revendication 13,
caractérisée en ce que
sur le second transporteur rectiligne (2) est placé un dispositif (4) d'inspection d'embouchure et/ou de filetage, tandis que sur le troisième transporteur (16) est monté un second dispositif déviateur (25) destiné à évacuer les bouteilles reconnues détériorées par le dispositif (4) d'inspection d'embouchure et/ou de filetage.
